**Europäisches Patentamt**

**European Patent Office**

**Office européen des brevets**

(11) Veröffentlichungsnummer: **0 000 190**
**A1**

# (12) EUROPÄISCHE PATENTANMELDUNG

(21) Anmeldenummer: **78100230.8**

(22) Anmeldetag: **23.06.78**

(51) Int. Cl.²: **A 61 K 31/135, A 61 K 9/06**

(30) Priorität: **01.07.77 DE 2729786**

(43) Veröffentlichungstag der Anmeldung:
**10.01.79 Patentblatt 79/1**

(84) Benannte Vertragsstaaten:
**DE FR GB NL**

(71) Anmelder: **Dr. Karl Thomae GmbH,**
**Postfach 720,**
**D-7950 Biberach (Riss) (DE)**

(72) Erfinder: **Keck, Johannes, Dipl.-Chem., Dr.,**
**Talfeldstrasse 27,**
**D-7950 Biberach 1 (DE)**

(72) Erfinder: **Püschmann, Sigfrid, Dr.,**
**Göserweg 8,**
**D-7950 Biberach 1 (DE)**

(72) Erfinder: **von Seefeld, Heide Angela, Dr.,**
**Probststrasse 15,**
**D-7950 Biberach 1 (DE)**

(72) Erfinder: **Papendick, Uwe, Dr.,**
**Richard-Wagner-Strasse 6,**
**D-7951 Ummendorf (DE)**

(72) Erfinder: **Renovanz, Hans-Dietrich, Dr.,**
**Ranzweg 21,**
**D-7950 Biberach 1 (DE)**

(54) **Lokalanästhetisch wirksame Lösungen.**

(57) Gegenstand der vorliegenden Anmeldung ist die neue Verwendung von 2-Amino- 3,5-dibrom-N- (trans-4 hydroxycyclohexyl)- benzylamin und dessen Säureadditionssalze als Lokalanästhetikum und die hierfür geeigneten Lösungen.

**EP 0 000 190 A1**

Croydon Printing Company Ltd.

Beschreibung:

Die Verbindung 2-Amino-3,5-dibrom-N-(trans-4-hydroxycyclohexyl)-benzylamin (NA-872) und deren Säureadditionssalze werden erstmals in der Belgischen Patentschrift 698 244 beschrieben, hierbei werden als pharmakologische Eigenschaften neben einer Beeinflussung der peripheren Atmungsfunktion insbesondere die antipyretische, hustenstillende und sekretolytische Wirkung genannt.

Ferner ist aus der Belgischen Patentschrift 827 466 die Antiulcus-Wirkung von 2-Amino-3,5-dibrom-N-(trans-4-hydroxycyclohexyl)-benzylamin und dessen Säureadditionssalzen bekannt.

Überraschenderweise wurde nun gefunden, daß Lösungen, die gegebenenfalls in Kombination mit einem Fluoreszensfarbstoff wie Fluorescein 2-Amino-3,5-dibrom-N-(trans-4-hydroxy-cyclohexyl)-benzylamin oder dessen physiologisch verträgliche Säureadditionssalze mit anorganischen und organischen Säuren enthalten, eine vorzügliche kurzfristige lokalanästhetische Wirkung aufweisen, wie sie für kurzfristige Augenuntersuchungen oder bei kurz andauernden diagnostischen oder kleineren therapeutischen Manipulationen an Schleimhäuten erforderlich ist. Die Wirkungsdauer beträgt hierbei längstens 20 - 25 Minuten.

Die erfindungsgemäßen Lösungen erhält man durch Lösen von 2-Amino-3,5-dibrom-N-(trans-4-hydroxycyclohexyl)-benzylamin oder dessen physiologisch verträglichen Säureadditionssalzen mit anorganischen und organischen Säuren in einem geeigneten wässrigen Träger in einer Konzentration von 0,5 - 4 %, vorzugsweise jedoch 1,0 - 2,5 % gegebenenfalls unter Hinzufügung eines Fluoreszensfarbstoffes wie Fluorescein.

Die erfindungsgemäßen lokalanästhetisch wirksamen Lösungen weisen beispielsweise als Augentropfen eine lokalanästhetische Wirkung auf, wie sie insbesondere für kurzfristige Augenuntersuchungen, z.B. bei der Tonometrie erwünscht ist. Die Tonometrie, besonders die Applanationstonometrie, dient der Messung des intraokularen Druckes, insbesondere bei der laufenden Kontrolle von Glaukom-Patienten. Die im Handel befindlichen Lokalanästhetika für kurzfristige Augenuntersuchungen weisen eine zu lange Wirkungsdauer auf, was dazu führt, daß die Wirkung noch nicht abgeklungen ist, wenn der Patient wieder auf die Straße kommt und damit der Möglichkeit eines unbemerkten Eindringens von Staub und anderen Fremdkörpern zwischen Lidern und Hornhaut ausgesetzt ist, wodurch Verletzungen des Hornhaut-Epithels hervorgerufen werden, außerdem muß wegen der beim Glaukom-Patienten häufigen Anwendung beim Einsatz der bisher gebräuchlichen Lokalanästhetika mit allergischen Reaktionen gerechnet werden (siehe Therapiewoche 26, 8640 - 8643 (1976)).

Ferner sind die erfindungsgemäßen lokalanästhetisch wirksamen Lösungen als kurzfristig wirkende Schleimhaut-Anästhetika, wie sie für den Bereich des Mundes oder des Rachens erwünscht sind, geeignet. Bei ihrer Anwendung entfällt die mit einem länger wirkende Lokalanästhetikum einhergehende Belästigung des Patienten, nämlich die Geschmacksbeeinträchtigung, das pelzige Gefühl oder die Schluckbeschwerden durch Ausschalten des Schluckreflex-Mechanismus

Die überlegene kurzfristige lokalanästhetische Wirkung der neuen Lösungen wurde im Vergleich zu Proxymetacain (0,5%ige 2-Diäthyl-aminoäthyl-3-amino-4-propoxybenzoesäure-hydrochlorid-Lösung = Chibro ®-Kerakain) am Kaninchen wie folgt geprüft:

Den Versuchstieren, die nach Spaltlampen-Untersuchung keine Schädigung des Cornea-Epithels zeigten und klinisch keine konjunktivale Entzündung aufwiesen, wurden jeweils 2 Tropfen der entsprechenden Augentropfen appliziert. Die Sensibilität der zentralen Cornea ist eine Minute nach Applikation durch Berührungsreiz, das gesamte Hornhautepithel und die Sensibilität 5, 10, 15 und 30 Minuten nach Applikation beurteilt worden. Der Untersucher, der die Sensibilität und den Hornhaut-Epithel-Status beurteilte, war hierbei nicht darüber informiert, welches Auge jeden Tieres mit Chibro $^{®}$ -Kerakain behandelt war.

Die nachfolgende Tabelle enthält die aufgefundenen Versuchsergebnisse:

| Tierauge | Medikation | 1 | | 5 | | 10 | | 15 | | 30 Minuten | |
|---|---|---|---|---|---|---|---|---|---|---|---|
| | | A | HH | A | HH | A | HH | A | HH | A | HH |
| rechts | 0,5 % NA 872 | ++ | | + | o.B. | o | o.B. | o | o.B. | o | o.B. |
| links | Kerakain | ++ | | ++ | o.B. | ++ | o.B. | ++ | o.B. | o | o.B. |
| rechts | Kerakain | ++ | | ++ | ++ | ++ | o.B. | (+) | o.B. | o | o.B. |
| links | 1,0 % NA 872 | ++ | | + | o.B. | + | o.B. | (+) | o.B. | o | o.B. |
| rechts | 2,0 % NA 872 | ++ | | ++ | o.B. | + | o.B. | o | o.B. | o | o.B. |
| links | Kerakain | ++ | | ++ | + | ++ | + | + | (+) | + | |

Die Tabelle zeigt: Erhobener Anästhesie-Befund (A) und Hornhaut-Befund in Minuten nach Applikation der Augentropfen.

++ starke Anästhesie bzw. deutliche Epithel-Veränderungen

+ deutliche Anästhesie bzw. kleine Epithel-Veränderungen

(+) Anästhesie eindeutig feststellbar bzw. kaum wahrnehmbare Epithel-Veränderungen

o keine Wirkung

Die erfindungsgemäßen neuen lokalanästhetisch wirksamen Lösungen (NA 872) weisen somit eine kürzere lokalanästhetische Wirkung als die Vergleichslösung auf, außerdem eine geringere Nebenwirkung auf das Epithel.

Die oben aufgezeigte Wirkung konnte unter Heranziehung des Aesthsiometers nach Cochet und Bonnet am menschlichen Auge mit Tropf- bzw. bei der Anwendung als Spray-Applikation mit Hilfe des Pump-Dosier-Ventils an der Mund- bzw. Rachenschleimhaut bestätigt werden. Bei einer Konzentration von 0,5 bis 1 % NA 872 und der Applikation von 1 bis 2 Tropfen pro Auge tritt die lokalanästhetische Wirkung innerhalb von 30 bis 60 Sekunden ein, die Wirkungsdauer betrug 15 bis 30 Minuten, bzw. bei der Applikation einer 2%igen NA 872-Lösung mittels 3-8 Hüben des Pump-Dosier-Ventils an der Mund- bzw. Rachenschleimhaut des Menschens konnte an 6 Probanden eine leichte Anästhesie nachgewiesen werden, die Wirkungsdauer betrug 15 bis 25 Minuten.

Ergänzend sei hier erwähnt, daß die Substanz 2-Amino-3,5-dibrom-N-(trans-4-hydroxycyclohexyl)-benzylamin und dessen physiologisch verträgliche Säureadditionssalze mit anorganischen und organischen Säuren eine geringe akute Toxizität aufweist, beispielsweise als Hydrochlorid eine $LD_{50}$ von 2 720 mg/kg p.o. an der Maus.

Die nachfolgenden Beispiele sollen die Erfindung näher erläutern:

## Beispiel 1

Augentropfen mit 0,5 % 2-Amino-3,5-dibrom-N-(trans-4-hydroxycyclo-hexyl)-benzylamin-hydrochlorid

Zusammensetzung:

| | | |
|---|---|---|
| Wirksubstanz | 0,5 | g |
| Citronensäure | 0,1 | g |
| $Na_2HPO_4$ x 2 $H_2O$ | 0,2 | g |
| Natriumchlorid | 0,8 | g |
| Alkyl-benzyl-dimethylammoniumchlorid | 0,02 | g |
| hochmolekulare Methylcellulose | 0,1 | g |
| Aqua dest. ad | 100 | ml |

## Herstellung:

In der Hälfte des Wassers werden unter schwachem Erwärmen und Rühren Citronensäure, Wirksubstanz, $Na_2HPO_4$ x 2$H_2O$ und Natrium-chlorid gelöst (Lösung I).

In der anderen Hälfte des Wassers werden unter Eiskühlung und Rühren Alkyl-benzyl-dimethylammoniumchlorid und Methylcellulose gelöst und über Nacht quellen gelassen (Lösung II).

Lösung I und II werden vereinigt und durch ein Membranfilter fil-triert.

## Beispiel 2

Augentropfen mit 2 % 2-Amino-3,5-dibrom-N-(trans-4-hydroxycyclo-hexyl)-benzylamin-hydrochlorid

Zusammensetzung:

| | | |
|---|---|---|
| Wirksubstanz | 2,0 | g |
| Citronensäure | 0,1 | g |
| $Na_2HPO_4$ x 2 $H_2O$ | 0,2 | g |
| Sorbit | 2,0 | g |

| | | |
|---|---|---|
| Polyvinylalkohol | 1,4 | g |
| Alkyl-benzyl-dimethylammoniumchlorid | 0,03 | g |
| Aqua dest. | ad | 100 ml |

## Herstellung:

In der Hälfte des Wassers werden unter schwachem Erwärmen und Rühren Citronensäure, Wirksubstanz und $Na_2HPO_4$ x $2H_2O$ nacheinander gelöst (Lösung I).

In der anderen Hälfte des Wassers werden bei 90°C und unter Rühren Sorbit, Polyvinylalkohol und Alkyl-benzyl-dimethylammoniumchlorid gelöst, anschließend wird auf Raumtemperatur abgekühlt und über Nacht quellen gelassen (Lösung II).

Lösung I und II werden vereinigt und durch ein Membranfilter filtriert.

## Beispiel 3

Augentropfen mit 0,5 % 2-Amino-3,5-dibrom-N-(trans-4-hydroxy-cyclohexyl)-benzylamin-hydrochlorid

Zusammensetzung:

| | | |
|---|---|---|
| Wirksubstanz | 0,5 | g |
| Borsäure | 2,5 | g |
| Borax | 0,3 | g |
| Alkyl-benzyl-dimethylammoniumchlorid | 0,004 | g |
| Aqua dest. | ad | 100 ml |

## Herstellung:

Borsäure wird in Wasser bei 60°C gelöst. Nach dem Abkühlen auf Raumtemperatur gibt man hierzu nacheinander Borax, Wirksubstanz und Alkyl-benzyl-dimethylammoniumchlorid. Nach Quellen über Nacht wird über ein Membranfilter filtriert.

Beispiel 4

Augentropfen mit 1,0 % 2-Amino-3,5-dibrom-N-(trans-4-hydroxy-cyclohexyl)-benzylamin-hydrochlorid

Zusammensetzung:

| | | |
|---|---|---|
| Wirksubstanz | 1,0 | g |
| Citronensäure | 0,1 | g |
| $Na_2HPO_4$ x 2 $H_2O$ | 0,2 | g |
| Natriumchlorid | 0,7 | g |
| Alkyl-benzyl-dimethylammoniumchlorid | 0,03 | g |
| hochmolekulare Methylcellulose | 0,1 | g |
| Aqua dest. ad | 100 | ml |

Herstellung:
analog Beispiel 1.

Beispiel 5

Augentropfen mit 2,0 % 2-Amino-3,5-dibrom-N-(trans-4-hydroxy-cyclohexyl)-benzylamin-hydrochlorid

Zusammensetzung:

| | | |
|---|---|---|
| Wirksubstanz | 2,0 | g |
| Citronensäure | 0,05 | g |
| $Na_2HPO_4$ | 0,5 | g |
| Sorbit | 2,0 | g |
| hochmolekulare Methylcellulose | 0,1 | g |
| Natriumchlorid | 0,3 | g |
| Phenyl-Hg-borat | 0,001 | g |
| Aqua dest. ad | 100 | ml |

Herstellung:

Lösung I wird analog Beispiel 1 hergestellt.

In der anderen Hälfte des Wassers werden unter Eiskühlung und Rühren Sorbit, Methylcellulose und Phenyl-Hg-borat gelöst und über Nacht quellen gelassen (Lösung II).

Lösung I und II werden vereinigt und durch ein Membranfilter filtriert.

## Beispiel 6

Auftropflösung mit 0,5 - 2,0 % 2-Amino-3,5-dibrom-N-(trans-4-hydroxycyclohexyl)-benzylamin-hydrochlorid

Zusammensetzung:

| | |
|---|---|
| Wirksubstanz | 0,5 - 2,0 g |
| Zitronensäure | 0,05 g |
| $Na_2HPO_4$ . 2 $H_2O$ | 0,1 g |
| Natriumchlorid | 0,8 - 0,6 g |
| Alkyl-benzyl-dimethylammoniumchlorid | 0,02 g |
| Aqua dest. ad | 100 ml |

Herstellung:

Die Lösung erfolgt in der Reihenfolge Zitronensäure, Wirksubstanz, $Na_2HPO_4$, Natriumchlorid und Alkyl-benzyl-dimethylammoniumchlorid in Wasser. Anschließend wird mit einem Membranfilter filtriert.

### Beispiel 7

Pinselung mit 0,5 - 2,0 % 2-Amino-3,5-dibrom-N-(trans-4-hydroxy-cyclohexyl)-benzylamin-hydrochlorid

Zusammensetzung:

| | |
|---|---|
| Wirksubstanz | 0,5 - 2,0 g |
| Zitronensäure | 0,05 g |
| $Na_2HPO_4$ . 2 $H_2O$ | 0,1 g |
| Sorbit | 1,0 g |
| Glycerin | 10,0 g |
| Alkyl-benzyl-dimethylammoniumchlorid | 0,03 g |
| Aqua dest. ad | 100 ml |

### Herstellung:

Die Substanzen werden in folgender Reihenfolge in der größeren Menge Wasser gelöst: Wirksubstanz, Zitronensäure, $Na_2HPO_4$, Alkyl-benzyl-dimethylammoniumchlorid, Sorbit und Glycerin. Anschließend wird filtriert.

### Beispiel 8

Lösung für Pumpdosierventilspray mit 0,5 - 2,0 % 2-Amino-3,5-dibrom-N-(trans-4-hydroxycyclohexyl)-benzylamin

Zusammensetzung:

| | |
|---|---|
| Wirksubstanz | 0,5 - 2,0 g |
| $Na_2HPO_4$ | 0,2 g |
| 1,2-Propylenglykol | 7,0 g |
| Alkyl-benzyl-dimethylammoniumchlorid | 0,03 g |
| Natriumchlorid | 0,6 - 0,4 g |
| Aqua dest. ad | 100 ml |

<u>Herstellung:</u>

Die Lösung wird in folgender Reihenfolge in der Hauptmenge Wasser durchgeführt: Zitronensäure, Wirksubstanz, $Na_2HPO_4$, Alkyl-benzyl-dimethylammoniumchlorid, Natriumchlorid und Propylenglykol. Danach wird mit Wasser aufgefüllt und druckfiltriert.

<u>Beispiel 9</u>

Dosier-Aerosol auf Treibgasbasis mit 0,5 - 2,0 % 2-Amino-3,5-dibrom-N-(trans-4-hydroxycyclohexyl)-benzylamin

Zusammensetzung:

| | |
|---|---|
| Wirksubstanz | 0,5 g |
| Sorbitantrioleat | 0,5 g |
| Isopropylmyristat | 1,5 g |
| Treibgasgemisch (11:12:114) ad | 100,0 g |

<u>Herstellung:</u>

Wirksubstanz, Sorbitantrioleat, Isopropylmyristat werden zusammen in einem Teil Treibgas 11 in der Kugelmühle gemahlen. Dieses Konzentrat wird in eine Kaltfüllanlage überführt, auf -40°C abgekühlt und mit dem Rest Treibgasgemisch aufgefüllt und in geeignete Aerosoldosen abgefüllt, sofort mit einem geeigneten Dosierventil versehen und verschlossen.

0000190

Dr. Karl Thomae GmbH
Case 5/716
Dr. Fl./Kp.

## Lokalanästhetisch wirksame Lösungen

**P a t e n t a n s p r ü c h e :**

1. Lokalanästhetisch wirksame Lösungen, enthaltend 2-Amino-3,5-dibrom-N-(trans-4-hydroxycyclohexyl)-benzylamin oder dessen Säureadditionssalze.

2. Lokalanästhetisch wirksame Lösungen gemäß Anspruch 1, dadurch gekennzeichnet, daß diese 2-Amino-3,5-dibrom-N-(trans-4-hydroxycyclohexyl)-benzylamin oder dessen Säureadditionssalze in einer Konzentration von 0,5 - 4 % enthalten.

3. Lokalanästhetische Augentropfen gemäß Anspruch 1 und 2, dadurch gekennzeichnet, daß diese als weiteren Hilfsstoff einen Fluoreszensfarbstoff wie Fluorescein enthalten.

4. Verwendung von 2-Amino-3,5-dibrom-N-(trans-4-hydroxycyclohexyl)-benzylamin oder dessen Säureadditionssalzen zur Herstellung von Lösungen mit einer lokalanästhetischen Wirkung.

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | A 61 K 31/135 <br> A 61 K 9/06 |
| X | CHEMICAL ABSTRACTS, <u>54</u>, 9106 bcd (1960) zitiert "Farmaco"(Pavia) Ed.sci. 14, 440-64 (1959) <br> * Zusammenfassung * | 1,2,4 | |
| X | <u>GB - 1 113 760</u> (BARNES-HIND LABORATORIES INC.) <br> * Seite 1, Zeilen 22-25 * | 3 | |

**RECHERCHIERTE SACHGEBIETE (Int. Cl.⁴)**

A 61 K 31/135
A 61 K 9/06

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort Den Haag | Abschlußdatum der Recherche 02-10-1978 | Prüfer BRINKMANN |
|---|---|---|

EPA form 1503.1